# EUROPEAN PATENT SPECIFICATION

(11) **EP 2 296 567 B1**
(45) Date of publication and mention of the grant of the patent: **12.03.2014**
(21) Application number: 09759565.6
(22) Date of filing: 05.06.2009
(51) Int. Cl.: A61B 17/70

(54) **APPARATUS FOR LOCKING A BAND**
GERÄT ZUR ARRETIERUNG EINES BANDS
APPAREIL DE BLOCAGE D'UNE SANGLE

(30) Priority: 06.06.2008 US 59543
(43) Date of publication of application: 23.03.2011
(73) Proprietor: Simpirica Spine, Inc., San Carlos, CA 94070 (US)
(72) Inventor: FIELDING, Louis, San Carlos CA 94070 (US); MALANDAIN, Hugues, Mountain View CA 94043 (US); BENNETT, Ian, San Francisco, CA 94109 (US); ALAMIN, Todd, Woodside CA 94062 (US)
(74) Representative: Clark, Jane Anne
(86) International application number: PCT/US2009/046492
(87) International publication number: WO 2009/149407

(56) References cited:
- WO-A2-2006/034423
- FR-A1- 2 704 745
- US-A- 5 415 658
- US-A1- 2002 072 753
- US-A1- 2002 116 013
- US-A1- 2004 097 985
- US-A1- 2004 117 017
- US-B1- 6 605 091

## Description

### BACKGROUND OF THE INVENTION

### 1. Field of the Invention.

The present invention generally relates to medical apparatus. More particularly, the present invention relates to orthopedic internal fixation and devices for restricting spinal flexion in patients having back pain, other spinal conditions, providing fracture fixation in long bone and trochanteric fractures or other orthopedic applications where a tether may be employed.

A major source of chronic low back pain is discogenic pain, also known as internal disc disruption. Patients suffering from discogenic pain tend to be young, otherwise healthy individuals who present with pain localized to the back. Discogenic pain usually occurs at the discs located at the L4-L5 or L5-S1 junctions of the spine. Pain tends to be exacerbated when patients put their lumbar spines into flexion (i.e. by sitting or bending forward) and relieved when they put their lumbar spines into extension (i.e. by standing or arching backwards). Flexion and extension are known to change the mechanical loading pattern of a lumbar segment. When the segment is in extension, the axial loads borne by the segment are shared by the disc and facet joints (approximately 30% of the load is borne by the facet joints). In flexion, the segmental load is borne almost entirely by the disc. Furthermore, the nucleus shifts posteriorly, changing the loads on the posterior portion of the annulus (which is innervated), likely causing its fibers to be subject to tension and shear forces. Segmental flexion, then, increases both the loads borne by the disc and causes them to be borne in a more painful way. Discogenic pain can be quite disabling, and for some patients, can dramatically affect their ability to work and otherwise enjoy their lives.

Pain experienced by patients with discogenic low back pain can be thought of as flexion instability, and is related to flexion instability manifested in other conditions. The most prevalent of these is spondylolisthesis, a spinal condition in which abnormal segmental translation is exacerbated by segmental flexion. The methods and devices described should as such also be useful for these other spinal disorders or treatments associated with segmental flexion, for which the prevention or control of spinal segmental flexion is desired. Another application for which the methods and devices described herein may be used is in conjunction with a spinal fusion, in order to restrict motion, promote healing, and relieve pain postoperatively. Alternatively, the methods and devices described should also be useful in conjunction with other treatments of the anterior column of the spine, including kyphoplasty, total disc replacement, nucleus augmentation and annular repair. General orthopedic or surgical applications are envisioned where a tether, cable or tape may be employed. An example is tronchanteric fracture fixation in which a cerclage device is wrapped around the bone and is attached and tightened to facilitate fracture healing. Similarly, the device may also be used in conjunction with a cerclage device for the fixation of long bone fractures.

Patients with discogenic pain accommodate their syndrome by avoiding positions such as sitting, which cause their painful segment to go into flexion, and preferring positions such as standing, which maintain their painful segment in extension. One approach to reducing discogenic pain involves the use of a lumbar support pillow often seen in office chairs. Biomechanically, the attempted effect of the ubiquitous lumbar support pillow is also to maintain the painful lumbar segment in the less painful extension position.

Current treatment alternatives for patients diagnosed with chronic discogenic pain are quite limited. Many patients follow a conservative treatment path, such as physical therapy, massage, anti-inflammatory and analgesic medications, muscle relaxants, and epidural steroid injections, but typically continue to suffer with a significant degree of pain. Other patients elect to undergo spinal fusion surgery, which commonly requires discectomy (removal of the disk) together with fusion of adjacent vertebra. Fusion may or may not also include instrumentation of the affected spinal segment including, for example, pedicle screws and stabilization rods. Fusion is not usually recommended for discogenic pain because it is irreversible, costly, associated with high morbidity, and has questionable effectiveness. Despite its drawbacks, however, spinal fusion for discogenic pain remains common due to the lack of viable alternatives.

An alternative method, that is not commonly used in practice, but has been approved for use by the United States Food and Drug Administration (FDA), is the application of bone cerclage devices which can encircle the spinous processes or other vertebral elements and thereby create a restraint to motion. Physicians typically apply a tension or elongation to the devices that applies a constant and high force on the anatomy, thereby fixing the segment in one position and allowing effectively no motion. The lack of motion allowed after the application of such devices is thought useful to improve the likelihood of fusion performed concomitantly; if the fusion does not take, these devices will fail through breakage of the device or of the spinous process to which the device is attached. These devices are designed for static applications and are not designed to allow for dynamic elastic resistance to flexion across a range of motion. The purpose of bone cerclage devices and other techniques described above is to almost completely restrict measurable motion of the vertebral segment of interest. This loss of motion at a given segment gives rise to abnormal loading and motion at adjacent segments, which can lead eventually to adjacent segment morbidity.

Another solution involves the use of an elastic structure, such as tethers, coupled to the spinal segment. The elastic structure can relieve pain by increasing passive resistance to flexion while often allowing substantially unrestricted spinal extension. This mimics the mechanical effect of postural accommodations that patients already use to provide relief.

Spinal implants using tether structures are currently commercially available. One such implant couples adjacent vertebrae via their pedicles. This implant includes spacers, tethers and pedicle screws. To install the implant, selected portions of the disc and vertebrae bone are removed. Implants are then placed to couple two adjacent pedicles on each side of the spine. The pedicle screws secure the implants in place. The tether is clamped to the pedicle screws with set-screws, and limits the extension/flexion movements of the vertebrae of interest. Because significant tissue is removed and because of screw placement into the pedicles, the implant and accompanying surgical methods are highly invasive and the implant is often irreversibly implanted. There is also an accompanying high chance of nerve root damage. Where the tip of the set-screw clamps the tethers, the tethers are abraded and particulate wear debris is generated.

Other implants employing tether structures couple adjacent vertebrae via their processes instead. These implants include a tether and a spacer. To install the implant, the supraspinous ligament is temporarily lifted and displaced. The interspinous ligament between the two adjacent vertebrae of interest is then permanently removed and the spacer is inserted in the interspinous interspace. The tether is then wrapped around the processes of the two adjacent vertebrae, through adjacent interspinous ligaments, and then mechanically secured in place by the spacer or also by a separate component fastened to the spacer. The supraspinous ligament is then restored back to its original position. Such implants and accompanying surgical methods are not without disadvantages. These implants may subject the spinous processes to frequent, high loads during everyday activities, sometimes causing the spinous processes to break or erode. Furthermore, the spacer may put a patient into segmental kyphosis, potentially leading to long-term clinical problems associated with lack of sagittal balance. The process of securing the tethers is often a very complicated maneuver for a surgeon to perform, making the surgery much more invasive. And, as previously mentioned, the removal of the interspinous ligament is permanent. As such, the application of the device is not reversible.

More recently, less invasive spinal implants have been introduced. Like the aforementioned implant, these spinal implants are placed over one or more pairs of spinous processes and provide an elastic restraint to the spreading apart of the spinous processes occurring during flexion. However, spacers are not used and interspinous ligaments are not permanently removed. As such, these implants are less invasive and may be reversibly implanted. The implants typically include a tether and a securing mechanism for the tether. The tether may be made from a flexible polymeric textile such as woven polyester (PET) or polyethylene; multi-strand cable, or other flexible structure. The tether is wrapped around the processes of adjacent vertebrae and then secured by the securing mechanism. The securing mechanism may involve the indexing of the tether and the strap, e.g., the tether and the securing mechanism include discrete interfaces such as teeth, hooks, loops, etc. which interlock the two. Highly forceful clamping may also be used to press and interlock the tether with the securing mechanism. Many known implementations clamp a tether with the tip of a set-screw, or the threaded portion of a fastener. However, the mechanical forces placed on the spinal implant are unevenly distributed towards the specific portions of the tether and the securing mechanism which interface with each other. These portions are therefore typically more susceptible to abrasion, wear, or other damage, thus reducing the reliability of these spinal implants as a whole. Other known methods use a screw or bolt to draw other components together to generate a clamping force. While these methods may avoid the potentially damaging loads, the mechanical complexity of the assembly is increased by introducing more subcomponents.

For the aforementioned reasons, it would be desirable to provide improved methods and apparatuses to secure the tethers of such spinal implants together. In particular, such methods and apparatuses should be less invasive and should enable the tether to be more easily, reversibly, repeatably and reliably secured to an implant by a surgeon, in a surgery setting.

### 2. Description of the Background Art.

Patents and published applications of interest include: U.S. Patent Nos. 3,648,691; 4,643,178; 4,743,260; 4,966,600; 5,011,494; 5,092,866; 5,116,340; 5,180,393; 5,282,863; 5,395,374; 5,415,658; 5,415,661; 5,449,361; 5,456,722; 5,462,542; 5,496,318; 5,540,698; 5,562,737; 5,609,634; 5,628,756; 5,645,599; 5,725,582; 5,902,305; Re. 36,221; 5,928,232; 5,935,133; 5,964,769; 5,989,256; 6,053,921; 6,248,106; 6,312,431; 6,364,883; 6,378,289; 6,391,030; 6,468,309; 6,436,099; 6,451,019; 6,582,433; 6,605,091; 6,626,944; 6,629,975; 6,652,527; 6,652,585; 6,656,185; 6,669,729; 6,682,533; 6,689,140; 6,712,819; 6,689,168; 6,695,852; 6,716,245; 6,761,720; 6,835,205; 7,029,475; 7,163,558; Published U.S. Patent Application Nos. US 2002/0151978; US 2004/0024458; US 2004/0106995; US 2004/0116927; US 2004/0117017; US 2004/0127989; US 2004/0172132; US 2004/0243239; US 2005/0033435; US 2005/0049708; 2005/0192581; 2005/0216017; US 2006/0069447; US 2006/0136060; US 2006/0240533; US 2007/0213829; US 2007/0233096; Published PCT Application Nos. WO 01/28442 A1; WO 02/03882 A2; WO 02/051326 A1; WO 02/071960 A1; WO 03/045262 A1; WO2004/052246 A1; WO 2004/073532 A1; and Published Foreign Application Nos. EP0322334 A1; and FR 2 681 525 A1. The mechanical properties of flexible constraints applied to spinal segments are described in Papp et al. (1997) Spine 22:151-155; Dickman et al. (1997) Spine 22:596-604; and Garner et al. (2002) Eur. Spine J. S186-S191; A1 Baz et al. (1995) Spine 20, No. 11, 1241-1244; Heller, (1997) Arch. Orthopedic and Trauma Surgery, 117, No. 1-2:96-99; Leahy et al. (2000) Proc. Inst. Mech. Eng. Part H: J. Eng. Med. 214, No. 5: 489-495; Minns et al., (1997) Spine 22 No. 16:1819-1825; Miyasaka et al. (2000) Spine 25, No. 6: 732-737; Shepherd et al. (2000) Spine 25, No. 3: 319-323; Shepherd (2001) Medical Eng. Phys. 23, No. 2: 135-141; and Voydeville et al (1992) Orthop Traumatol 2:259-264.

WO 2006/034423 describes an implant comprising a spacer for defining a minimum space between adjacent spinous processes, a distraction guide for piercing and distracting an interspinous ligament during implantation and a binder for limiting or preventing flexion motion of the targeted motion segment. The binder can be secured to a brace associated with the implant during implantation by a capture device. The capture device may include a fixed piece extending from the brace and a slidable piece associated with the fixed piece. A fastener can be rotated to pinch the binder between the slidable piece and the wall of the brace, securing the binder.

### BRIEF SUMMARY OF THE INVENTION

The present invention is set out in the appended claims. Described herein are fastening mechanisms and methods for releasably locking a tether for restricting flexion of at least one spinal segment. More particularly, the provided fastening mechanisms and methods relate to improvements to the methods and devices of deploying and implanting spinal implants for the treatment of discogenic pain and other conditions, such as degenerative spondylolisthesis. Specifically, such deployment and implantation methods are made less invasive and more reliable and reversible by the provided fastening mechanisms and methods.

Described herein is a device for restricting flexion of a spinal segment. The device includes a constraint device having a tether structure and a compliance member. The constraint device is adapted to be coupled with adjacent spinous processes or a spinous process and a sacrum. The constraint device is also adapted to provide a force resistant to flexion of the spinal segment. A locking mechanism is coupled with the constraint device and includes a clamp body and a fastener element. The clamp body has a fastener aperture and a tether aperture. The fastener aperture is sized to receive the fastener element, and the tether aperture is sized to receive the tether structure. A portion of the tether structure is disposed in the tether aperture and the fastener aperture, and the fastener element is disposed in the fastener aperture such that the tether structure is captured between the fastener element and the clamp body.

The constraint may have a dimension that is adjustable to allow tightening over the spinous processes or spinous process and sacrum when the spinal segment is in a neutral position. The constraint device may provide an elastic resistance to flexion of the spinal segment beyond the neutral position in the range from 7.5 N/mm to 20 N/mm. The clamp body is coupled with the constraint. The fastener element may be disposed at least partially in the fastener aperture. The tether is captured between the fastener element and the clamp body.

In many embodiments, the constraint may provide an elastic resistance to extension beyond the neutral position below 3 N/mm. In some embodiments, the elastic resistance may be below 0.5 N/mm. In some embodiments, the constraint is pre-tensioned to provide an initial resistive force to flexion which must be overcome prior to initiating deformation. The initial resistive force may be in the range from 5N to 25N.

In many embodiments, the constraint may comprise a superior tether structure adapted to couple to the superior spinous process, an inferior tether structure adapted to couple to the inferior spinous process or sacrum, and at least one compliance member. The tether structures are substantially non-distensible and the compliance member provides the resistance to flexion. In some embodiments, the device may comprise at least two compliance members arranged to lie on either side of a midline when the tether structures are placed over the spinous processes or spinous process and sacrum.

In various embodiments, the tether or tether aperture may include various features. The tether aperture may comprise a rectangular shaped slot. The tether may enter the tether aperture without deformation thereof. The tether may be deformed in order to be inserted into the tether aperture. In some embodiments, the tether may have a width and the tether aperture may have a width smaller than the tether width. The tether enters the tether aperture in a first plane and the tether exits the tether aperture in a second plane generally transverse to the first plane.

In some embodiments, the fastener element comprises a screw threadably engaged with the fastener aperture. The screw may comprise a head having a diameter larger than the aperture diameter. The tether may be captured between the screw head and the clamp body. A surface of the clamp body may support the screw head and prevent bending of the screw. The screw may comprise a head having surface features adapted to press into the tether when the screw is tightened. The screw may comprise a driver feature adapted to receive a tool to permit rotation of the screw. The driver feature may be, for example, a Phillips head, a slotted flat head, a Torx head, or a hex head. The fastener element may rotationally lock the tether in position relative to the clamp body.

In some embodiments, the fastener element comprises a position indicator adapted to provide visual, tactile, or audible feedback to an operator on the relative position of the fastener element with respect to the clamp body. The position indicator may comprise detents or calibration marks on either the fastener element or the clamp body and the indicator may be radiopaque to permit visualization under x-ray, fluoroscopy or other radiographic techniques.

Described herein is a method for releasably locking a tether.
The tether is advanced through a tether aperture in a clamp body. The fastener element is positioned in a fastener aperture in the clamp body. The tether enters the tether aperture in a first plane and then exits the clamp body in a second plane generally transverse to the first plane. The tether is captured between the clamp body and a surface of the fastener. The fastener element thereby releasably locks the tether in position relative to the clamp body. The clamp body may have additional surfaces that support the screw in reaction to bending or other loads that may be induced in the screw by the clamping action.

The provided method may further comprise various steps and/or features. The fastener element may comprise a screw and capturing the tether may comprise threadably engaging the screw with the fastener aperture. The fastener element may comprise a screw and rotating the screw in a first direction locks the tether in position while rotating the screw in a second direction opposite the first may unlock the tether. The indicator indicates the position of the fastener element relative to the clamp body. Advancing the tether through the tether aperture may comprise advancing the tether therethrough without deformation of the tether or the tether may be deformed in order to fit in the tether aperture. A position indicator indicating the position of the fastener element relative to the clamp body may be monitored.

Described herein is a method for releasably locking an orthopedic, surgical tether comprises advancing the tether through a tether aperture in a clamp body and positioning a fastener element having a head, in the clamp body. Thus, the tether is captured between the clamp body and the head of the fastener element thereby releaseably locking the tether in position relative to the clamp body.

These and other embodiments are described in further detail in the following description related to the appended drawing figures.

### BRIEF DESCRIPTION OF THE DRAWINGS

Fig. 1 is a schematic diagram illustrating the lumbar region of the spine.
Fig. 1A a schematic illustration showing a portion of the lumbar region of the spine taken along a sagittal plane.
Fig. 2 illustrates a spinal implant of the type described in US 2005/0216017A1.
Figs. 3A-3B illustrate additional tissue surrounding the spinous processes.
Figs. 4A-4M show an exemplary method of surgically implanting a spinal device.
Figs. 5A-5K show an exemplary fastening mechanism using a screw clamp.
Figs. 6A-6B illustrate the use of a tether and fastening mechanism for trochanteric fixation.

### DETAILED DESCRIPTION OF THE INVENTION

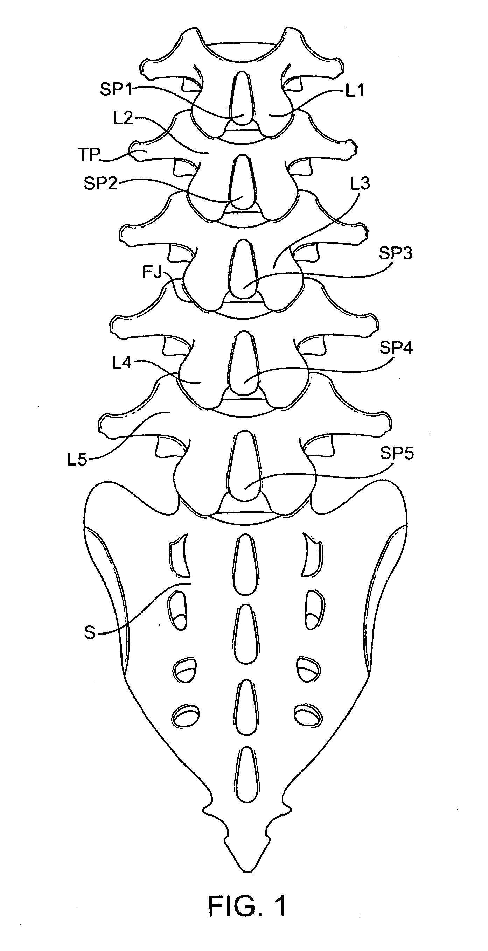

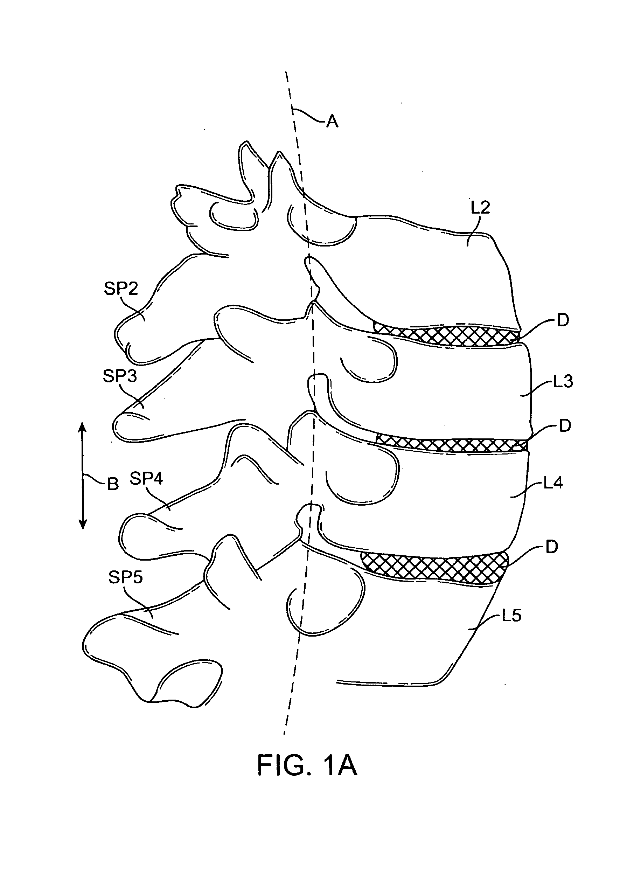

Fig. 1 is a schematic diagram illustrating the lumbar region of the spine including the spinous processes (SP), facet joints (FJ), lamina (L), transverse processes (TP), and sacrum (S). Fig. 1A is a schematic illustration showing a portion of the lumbar region of the spine taken along a sagittal plane and is useful for defining the terms "neutral position," "flexion," and "extension" that are often used in this disclosure.

As used herein, "neutral position" refers to the position in which the patient's spine rests in a relaxed standing position. The "neutral position" will vary from patient to patient. Usually, such a neutral position will be characterized by a slight curvature or lordosis of the spine where the spine has a slight anterior convexity and slight posterior concavity. In some cases, the presence of the constraint of the present invention may modify the neutral position, e.g. the device may apply an initial force which defines a "new" neutral position having some extension of the untreated spine. As such, the use of the term "neutral position" is to be taken in context of the presence or absence of the device. As used herein, "neutral position of the spinal segment" refers to the position of a spinal segment when the spine is in the neutral position.

Furthermore, as used herein, "flexion" refers to the motion between adjacent vertebrae in a spinal segment as the patient bends forward. Referring to Fig. 1A, as a patient bends forward from the neutral position of the spine, i.e. to the right relative to a curved axis A, the distance between individual vertebrae L on the anterior side decreases so that the anterior portion of the intervertebral disks D are compressed. In contrast, the individual spinous processes SP on the posterior side move apart in the direction indicated by arrow B. Flexion thus refers to the relative movement between adjacent vertebrae as the patient bends forward from the neutral position illustrated in Fig. 1A.

Additionally, as used herein, "extension" refers to the motion of the individual vertebrae L as the patient bends backward and the spine extends from the neutral position illustrated in Fig. 1A. As the patient bends backward, the anterior ends of the individual vertebrae will move apart. The individual spinous processes SP on adjacent vertebrae will move closer together in a direction opposite to that indicated by arrow B.

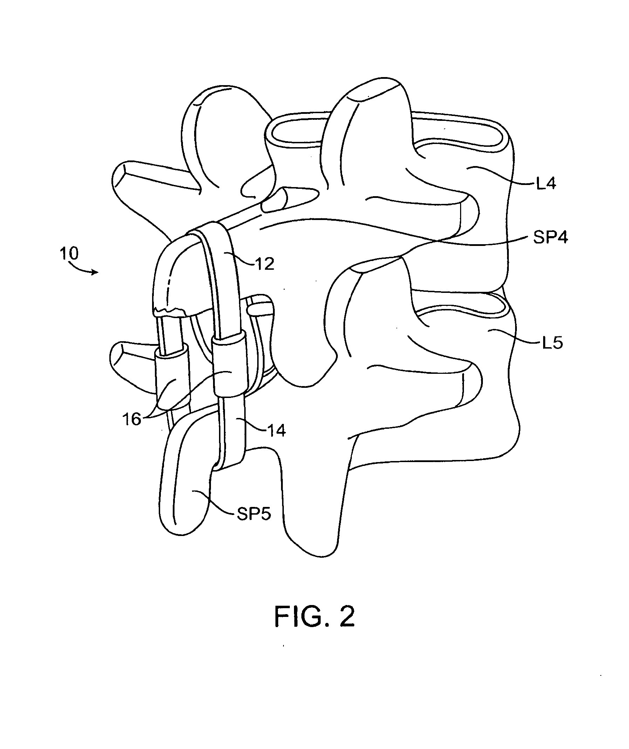

Fig. 2 shows a spinal implant of the type described in related U.S. Patent Publication No. 2005/02161017 A1,

As illustrated in Fig. 2, an implant 10 typically comprises an upper strap component 12 and a lower strap component 14 joined by a pair of compliance members 16. The upper strap 12 is shown disposed over the top of the spinous process SP4 of L4 while the lower strap 14 is shown extending over the bottom of the spinous process SP5 of L5. The compliance member 16 will typically include an internal element, such as a spring or rubber block, which is attached to the straps 12 and 14 in such a way that the straps may be "elastically" or "compliantly" pulled apart as the spinous processes SP4 and SP5 move apart during flexion. In this way, the implant provides an elastic tension on the spinous processes which provides a force that resists flexion. The force increases as the processes move further apart. Usually, the straps themselves will be essentially non-compliant so that the degree of elasticity or compliance may be controlled and provided solely by the compliance members 16.

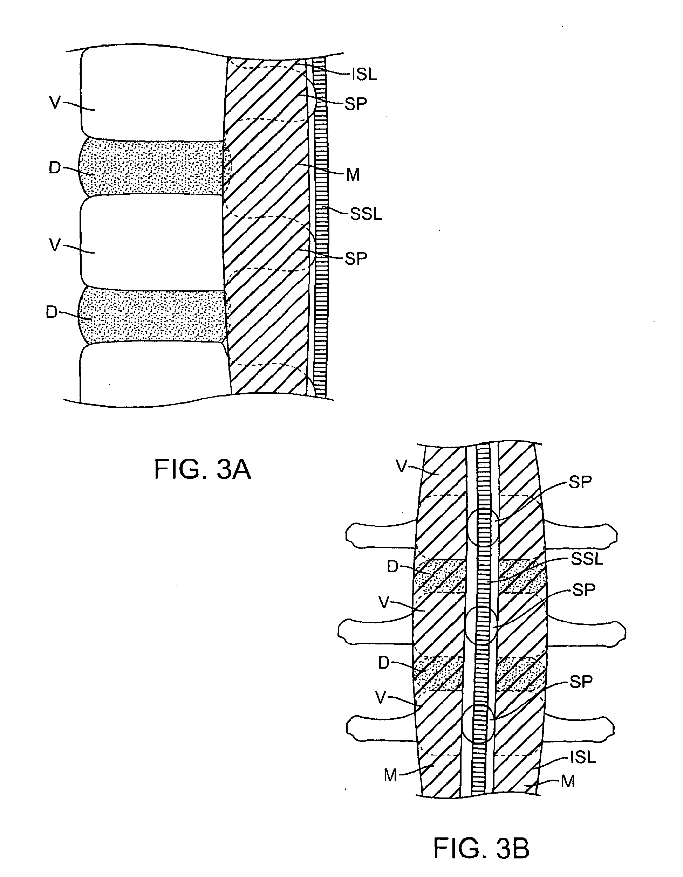

Fig. 3A is a side view of the lumbar region of the spine having discs D separating the vertebral bodies V. The supraspinous ligament SSL runs along the posterior portion of the spinous processes SP and the interspinous ligament ISL and multifidus tendon and muscle M run alongside of and attach to the spinous processes SP. Fig. 3B is a posterior view of Fig. 3A.

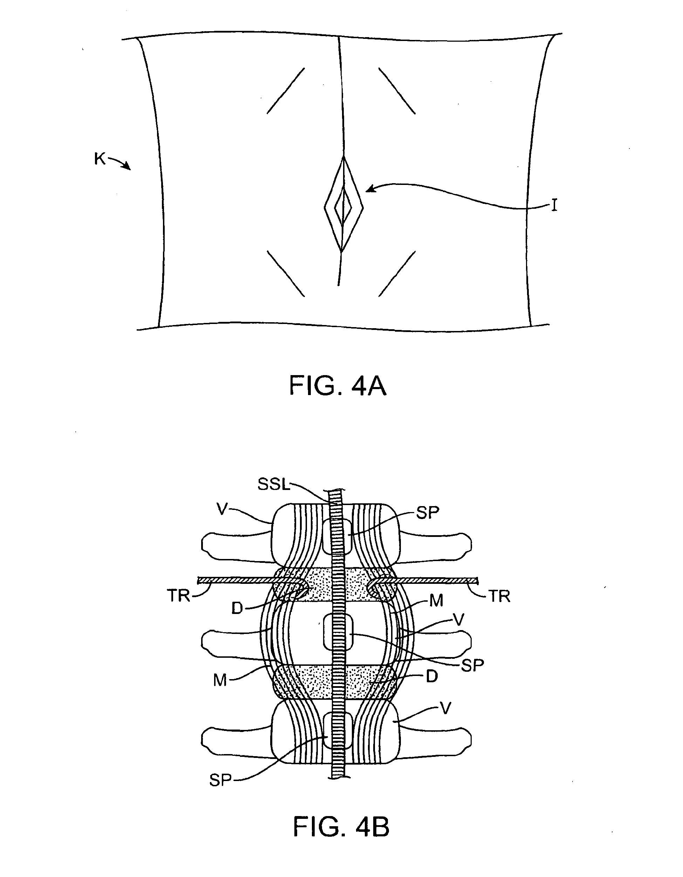

Figs. 4A-4M illustrate an exemplary surgical method of implanting a spinous process constraint such as the embodiment of Fig. 2. One of the first steps to surgically implant a spinal implant is to make an incision to access the spinal area of interest. Fig. 4A shows the lumbar region of back K after an incision I has been made through the patient's skin. Fig. 4B illustrates the lumbar region of the spine after the incision I has been made through the patient's skin. Multifidus muscle and tendon M have been retracted with retraction tools TR to expose the spinous processes.

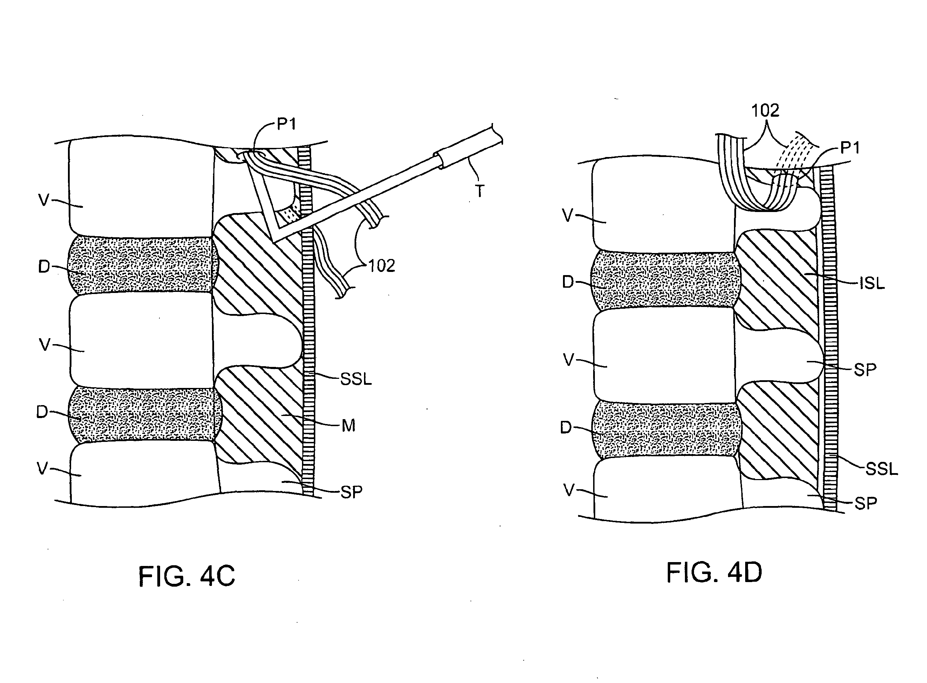

After the incision has been made, a piercing tool T having a sharp distal end may be used to access and pierce the interspinous ligament ISL while avoiding the supra spinous ligament SSL, creating an interspinous ligament perforation P1 superior of the first spinous process SSP of interest. This surgical approach is desirable since it keeps the supra spinous ligament intact and minimizes damage to the multifidus muscle and tendons and other collateral ligaments. As shown in Fig. 4C, from the right side of the spine, tool T accesses and pierces the interspinous ligament ISL adjacent of the first spinous process SSP of interest. The distal end of tool T is shown in dotted line. Alternatively, tool T may access and pierce the interspinous ligament ISL from the left side instead. The distal end of tool T is coupled with tether 102, parts of which are also shown in dotted line. In addition to accessing and piercing the interspinous ligament ISL, piercing tool T also advances or threads tether 102 through perforation P1. As shown in Fig. 4D, tool T is then removed, leaving tether 102 positioned through perforation P1. Multifidus tendon and muscle M is not shown in Figs. 4C and 4D so that other elements are shown more clearly.

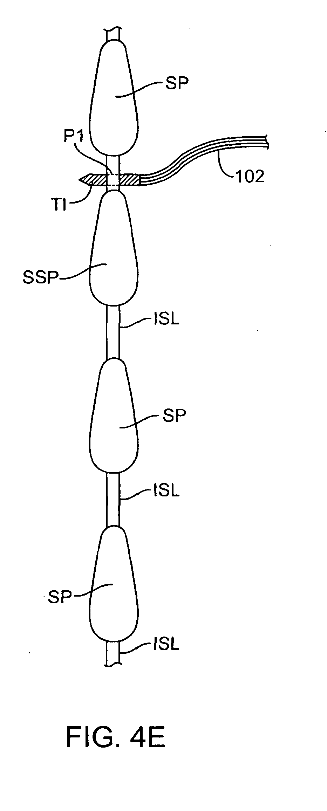

Fig. 4E is a posterior view of a section of the spine after the above steps have been performed. Often times, the distal tip TI of tool T is detachable. As shown in Fig. 4E, after tool T accesses and pierces the interspinous ligament ISL with distal tip TI, distal tip TI is detached from tool T and is left in place in perforation P1 (shown in dotted line) above the first spinous process SSP of interest. Tether 102 lags behind tip TI. In some cases, distal tip TI may fully pierce through interspinous ligament ISL. In these cases, distal tip TI has passed through the interspinous ligament ISL while a portion of tether 102 is left in place in perforation P1.

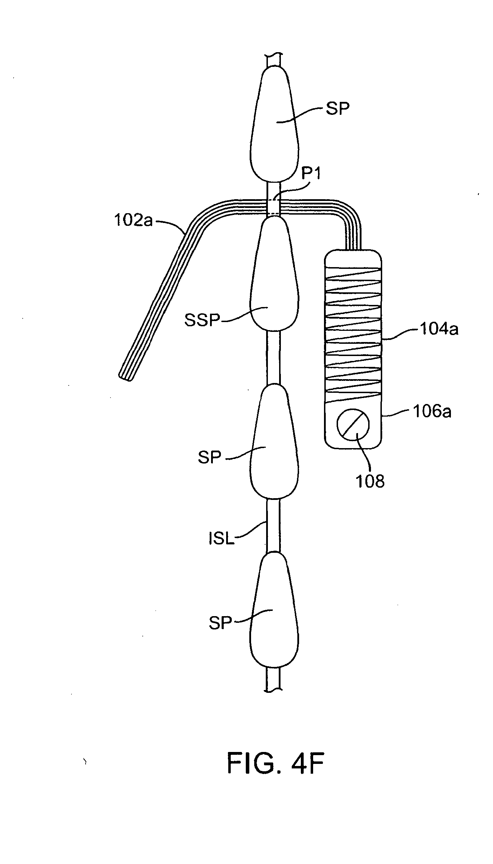

After tip TI or a portion of tether TH is left in place in perforation P1, another tool may couple with tip TI and pull tip TI such that it drags tether 102a and compliance element 104a to its appropriate position relative to the spine, as shown in Fig. 4F. Compliance element 104a is coupled to tether 102a and is used to provide a force resistive to flexion of spinous processes SP. Compliance element 104a includes a fastening mechanism or fastening element 106a and may further comprise a spring, a tensioning member, a compression member, or the like. Related compliance members are described in commonly owned U.S. Patent Application No. 12/106,103 (Attorney Docket No. 026398-000410US),

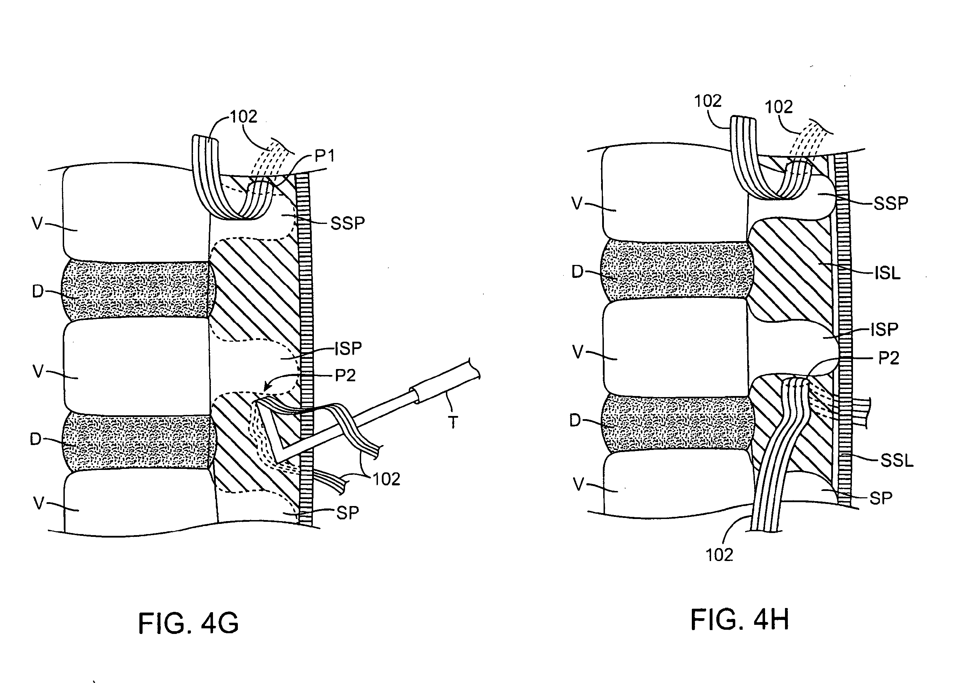

The steps of accessing the ISL, piercing the ISL, and threading tether 102 through a perforation are then repeated for the opposite, lateral side of the spine for an adjacent spinous process ISP, inferior of the first superior spinal process SSP of interest. As shown in Figs. 4G and 4H, tool T accesses the interspinous ligament from the left side of the spinal midline and pierces the interspinous ligament ISL, creating a second perforation P2 located inferior of a second spinous process of interest, labeled as inferior spinous process ISP. As shown in Fig. 4G, the inferior spinous process ISP of interest is directly adjacent to and inferior of the first superior spinous process SSP of interest. However, it is entirely possible to perform the described procedure starting with the inferior spinous process ISP first instead of the superior spinous process SSP, for example, perforation P2 may be created before perforation P1. It is also possible that there may be a gap of one or more spinous processes SP between the spinous processes of interest. Multifidus tendon and muscle M is not shown in Figs. 4G and 4H for clarity of the other shown elements.

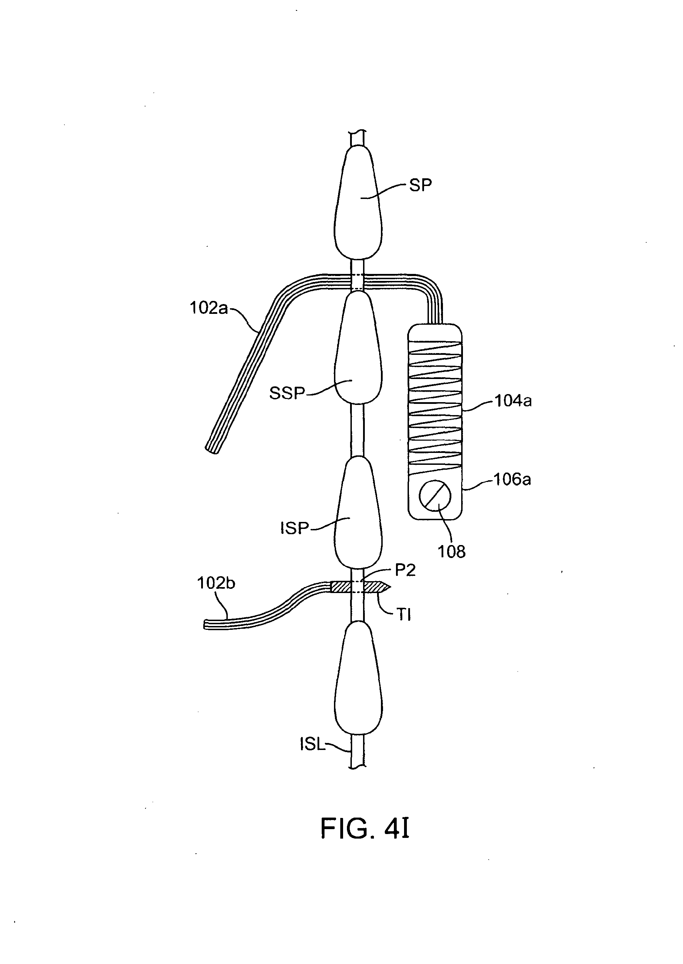

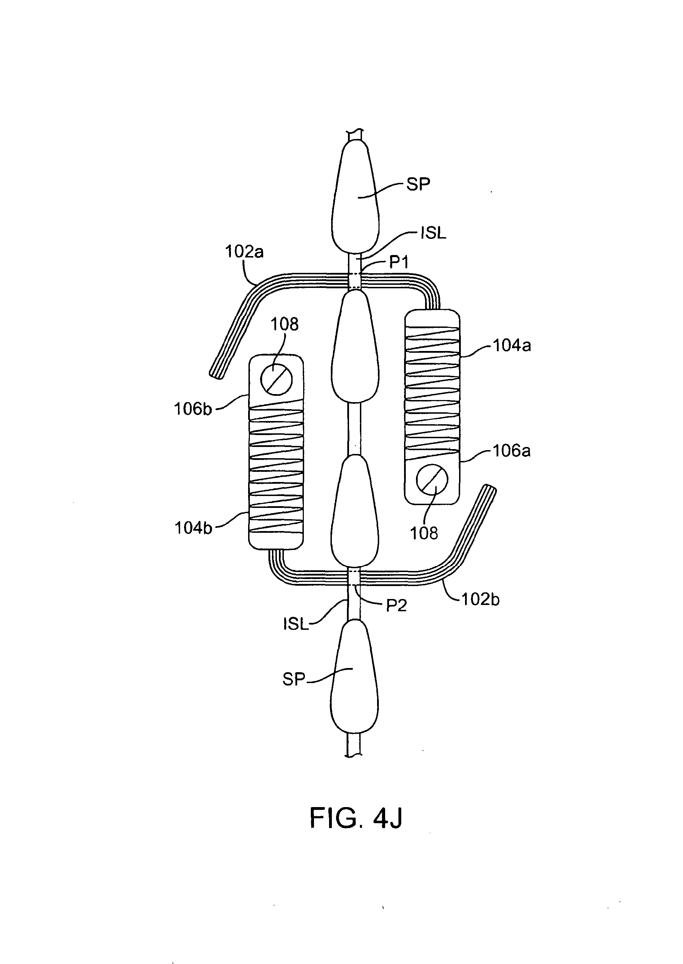

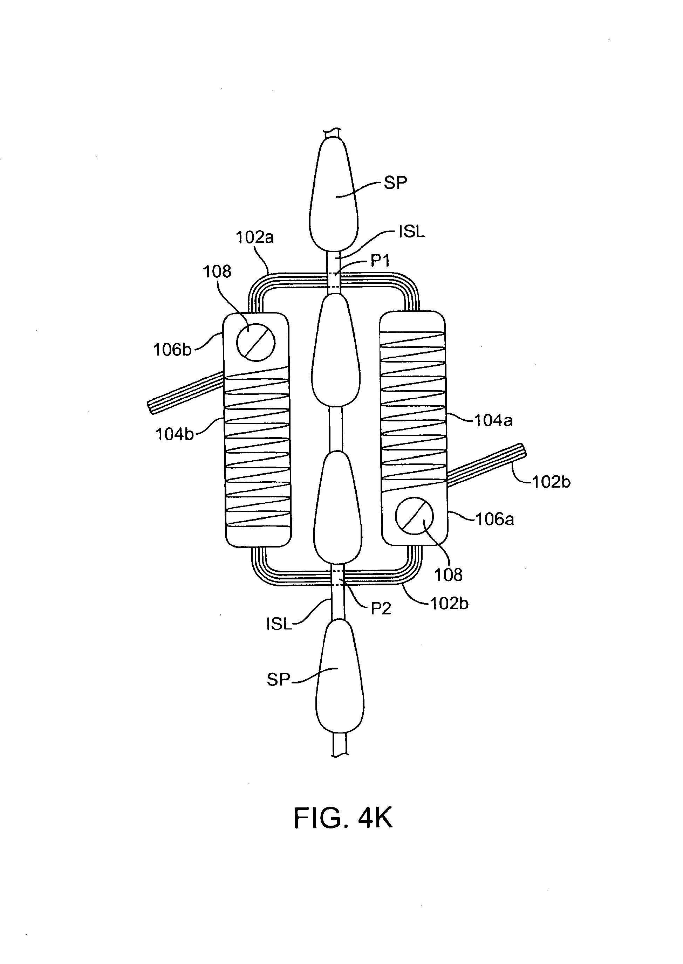

As shown in Figs. 4H, 4I and 4J, like with the steps shown in conjunction with the first piercing, tether 102b is pierced through perforation P2 and left in place along with distal tip TI of tool T (best seen in Fig. 4I). Another tool such as a pair of forceps, is then used to grasp distal tip TI to pull tether 102b and compliance element 104b in place relative to the spine, as shown in Fig. 4J. Opposing compliance members 104a and 104b on opposite sides of spinous processes SP are oriented in opposite directions. Each compliance element 104a, 104b is coupled with their respective tether 102a, 102b and has a respective fastening mechanism or fastening element 106a, 106b. Fastening mechanism 106a, 106b are configured to couple with the tether 102a, 102b of the opposing compliance member 104a, 104b. For example as shown in Fig. 4K, tether 102a is advanced through compliance member 104b and is coupled with fastening mechanism 106b while tether 102b is advanced through compliance member 104a and is coupled with fastening mechanism 106a. Except for their orientation, compliance members 104a and 104b are identical. One of skill in the art will appreciate that the tether may enter and exit the fastening mechanism in a number of different directions and configurations, and Fig. 4K merely is one exemplary embodiment.

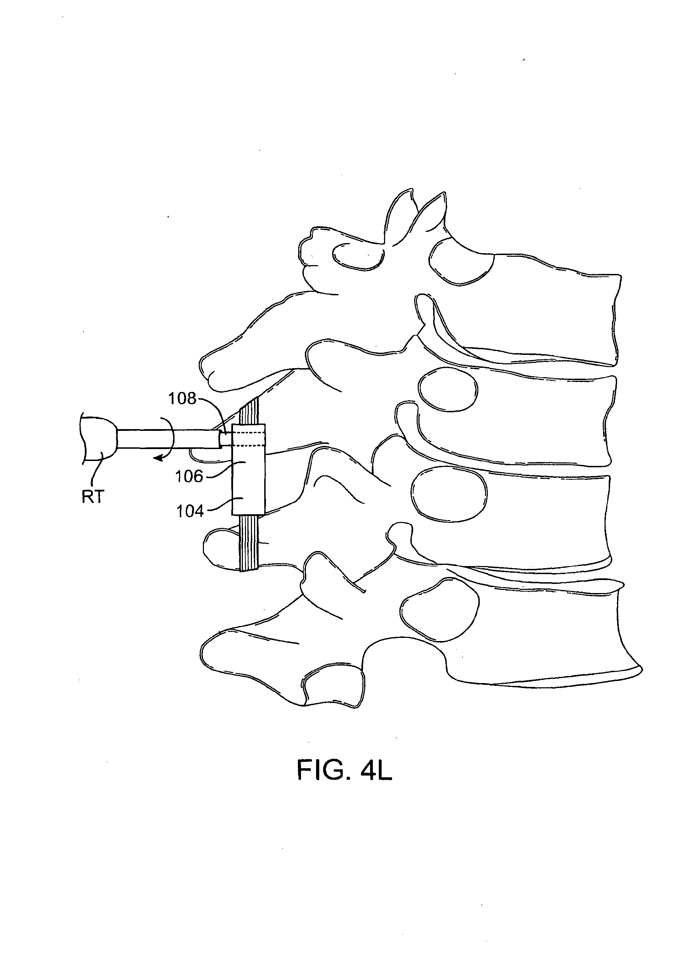

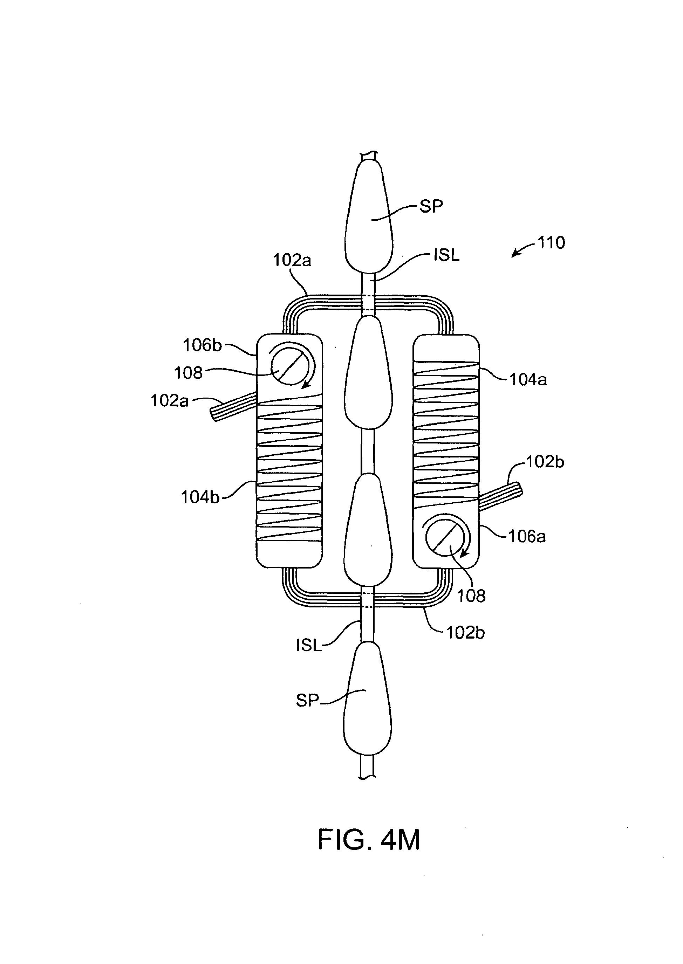

Fastening mechanism 106 may comprise a driver feature 108. As shown in Fig. 4L, the driver feature is adapted to receive a rotating driver tool RT. The driver feature may be a Phillips head, a slotted flat head, a Torx head, a hex head, or the like. Rotation of tool RT, which may be either clockwise or counter-clockwise, changes the configuration of fastening mechanism 106 so as to lock and secure tether 102 in place. This forms a continuous, multicomponent tether structure or constraint 110 which couples two spinous processes SP together, as shown in Fig. 4M. Compliance elements 104a, 104b are used to control flexion between spinous processes SP while tethers 102a, 102b and respective fastening mechanisms 106a, 106b contribute to coupling the spinous processes SP together. Depending on the location of the perforations P1 and P2 and the lengths of the compliance elements 104a, 104b, constraint 110 may couple more than two spinous processes SP together. In general, compliance elements 104a, 104b comprise spring-like elements which will elastically elongate as tension is applied through tethers 102a, 102b in an axis generally parallel to the spine. As the spinous processes or spinous process and sacrum move apart during flexion of the constrained spinal segment, the superior tether 102a and inferior tether 102b will also move apart. Compliance elements 104a, 104b each include spring-like elements which will elastically resist the spreading with a force determined by the mechanical properties of the spring-like element. Thus, constraint 110 provides an elastic resistance to flexion of the spinal segment beyond the neutral position. Constraint 110 is often configured to provide a resistance in the range from 7.5 N/mm to 20 N/mm but the resistance may be below 3 N/mm or even below 0.5 N/mm. Constraint 110 may also be adjustable in certain dimensions to allow tightening over the spinous processes or spinous process and sacrum when the spinal segment is in a neutral position. Other, related tether embodiments and joining methods are disclosed in U.S. Patent Application No. 12/106,103 (Attorney Docket No. 026398-000410US), U.S. Patent Publication No. 2008/0009866 (Attorney Docket No. 026398-000140US), U.S. Patent Publication No. 2008/0108993 (Attorney Docket No. 026398-000150US), U.S. Provisional Patent Application No. 60/936,897 (Attorney Docket No. 026398-000400US).

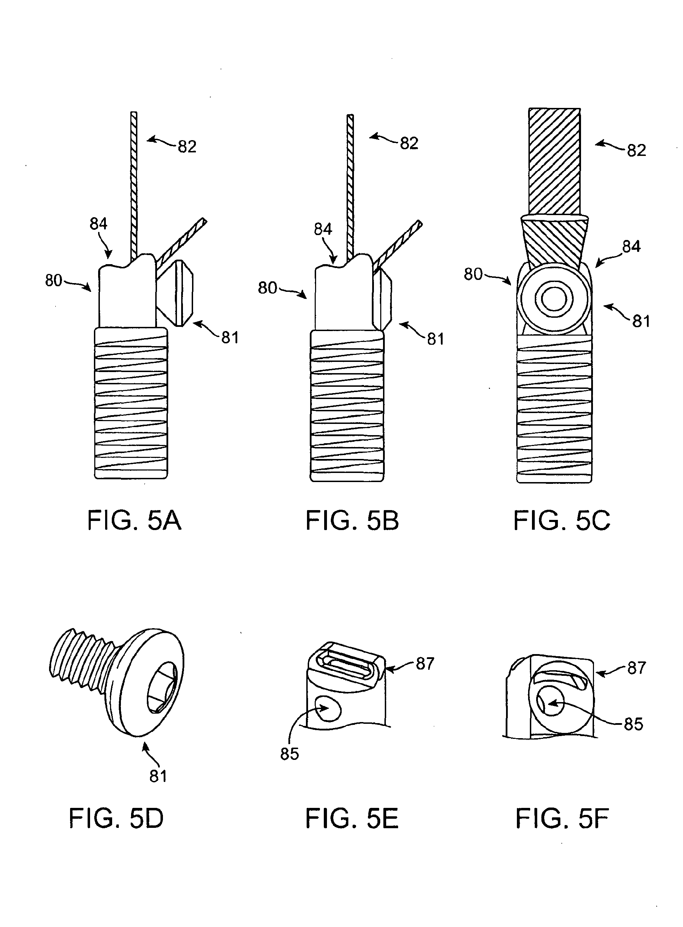

Tethers may be locked and secured in place relative to a compliance member using a screw clamp as seen in Figs. 5A-5K. The housing or clamp body 84 of compliance member 80 is configured to secure tether 82 in place with threaded screw 81 but is otherwise similar to compliance members 104a, 104b previously described. Like compliance member 104a, 104b, a pair of compliance members 80 and tethers 84 may be coupled together to form a constraint around spinous processes or a spinous process and sacrum. Housing 84 has a fastener aperture 85 and a tether aperture 87 as shown in the bottom view of Fig. 5E and the top view of Fig. 5F. Tether 82 is advanced through tether aperture 87 as shown in Fig. 5A. The shaft of screw 81 is positioned in fastener aperture 85 as also seen in Fig. 5A. Screw 81 often has threads which allow it to couple with fastener aperture 85. Screw 81 often has male threads while fastener aperture 85 has female threads. Male and female threads may be substituted with one another. Screw 81 preferably does not have sharp edges that will cut or otherwise damage the strap, either during fastening or in service. Screw 81 includes driver features such as a Philips head, a slotted flat head, a Torx head, a hex head, or the like adapted to receive a tool to permit rotation. Screw 81 is rotated and advanced through fastener aperture 85, capturing and clamping tether 82 between the clamping surface of the head of screw 81 and housing 84, as shown in the side view of Fig. 5B and top vie of Fig. 5C. Screw 81 may also has surface features adapted to press into the tether when the screw is tightened. For example, the clamping surface may have a specific texture like knurling that would increase or decrease retention strength for a given clamping force based on the smoothness or roughness of the texture. The head of screw 81 has a diameter larger than that of fastener aperture 85. Housing 84 and/or screw 81 may have position indicators such as detents or calibration marks adapted to provide visual, tactile or audible feedback on the relative position of screw 81 relative to housing 84. Rotation of screw 81 in an opposite direction loosens the fit of the tether between screw 81 and housing 85. Fig 5D illustrates the fastener screw 81 and Figs. 5E-5F show the apertures that receive the fastener and tether.

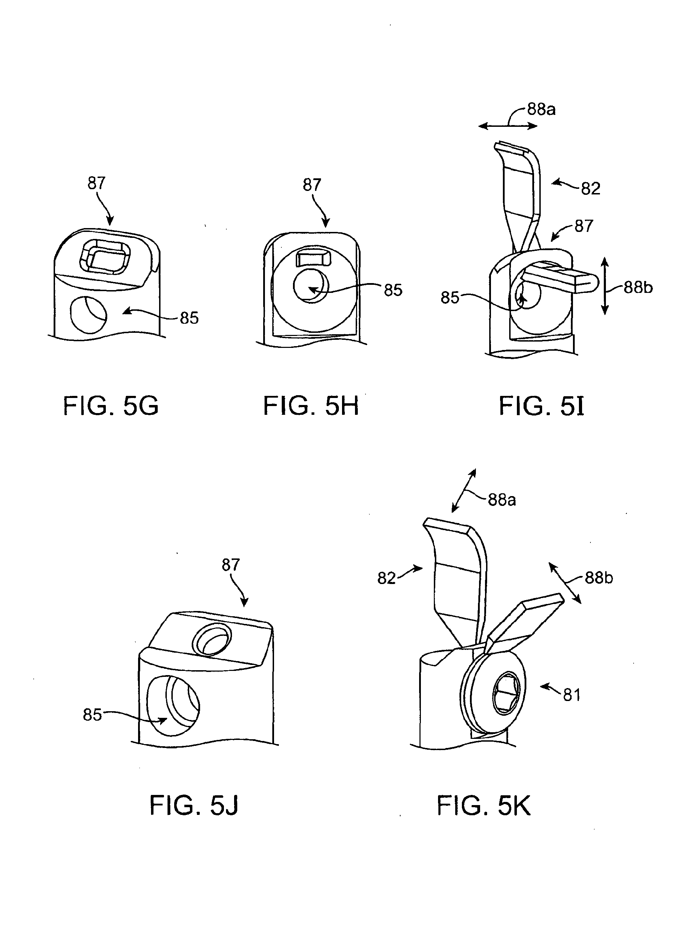

Tether aperture 87 is often a rectangular shaped slot, although one of skill in the art will appreciate that may geometries may be utilized. In Fig. 5G tether aperture 87 may be rectangular and have a width smaller than the width of the tether 82. Fig. 5H shows the opposite side of the tether aperture 87. Thus, as seen in Fig. 5I tether 82 will have to be deformed or folded to fit through the aperture. By deforming tether 82, tether 82 will be provided with additional flexibility in the directions shown by arrows 88a, 88b in Fig. 5I. This is advantageous since as tether 82 enters aperture 87, it generally is biased to flex only in the direction shown by arrow 88a. As tether enters aperture 87, it is folded and then is biased to flex in direction 88b which is transverse the direction of bias as tether 82 enters the aperture. Furthermore, tether 82 exits aperture 87 in a direction generally parallel to the direction that a screw of other fixing element enters aperture 85. This helps to ensure that the physician can easily grasp and adjust tether 82 since a pathway already exists for a fixing element to be engaged with aperture 85. Further details on this feature will be discussed below.

Tether aperture 87 may also be generally circular as shown in Fig. 5J. As seen in Fig. 5K, tether 82 may be deformed by twisting so that it enters the circular aperture in a first plane and then exits the circular aperture in a second plane generally transverse to the first plane. By twisting tether 82 as described, it is provided with additional flexibility in the directions shown in by arrows 88a, 88b. The deformation of tether 82 may also position the tether so that it can more easily be tensioned in a surgical procedure. Additionally, the deformation of tether 82 allows it to better conform to the anatomy. In Fig. 5I, the rectangular aperture causes tether 82 to fold and fan-out and in Fig. 5K, the circular aperture causes tether 82 to twist and fan-out. This enables the tail of the strap to be tensioned dorsally for ease of use, while the opposite, working end changes planes to contour medially and conform to the anatomy. Therefore, the tether can be tensioned from the same direction as application of the driver tool to lock the tether, thus permitting a less invasive procedure.

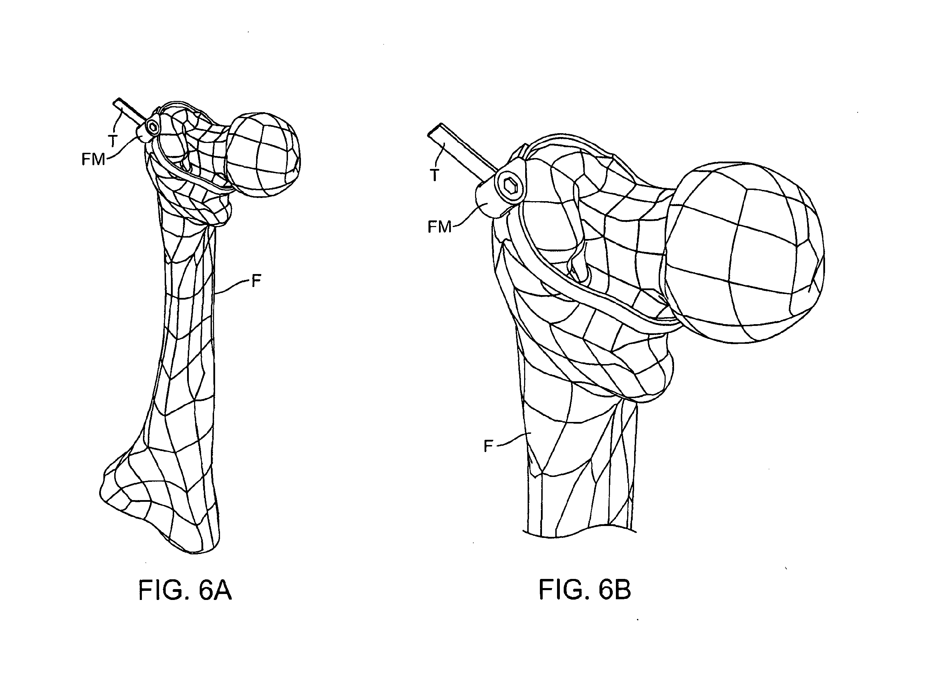

While the exemplary embodiments described above illustrate a fastening mechanism that is coupled with a spring-like compliance member, one will appreciate that the fastening mechanism may be used independently of a spring or other internal fixator. Other uses may include applications where a tether is secured with a knot, crimped or the like. These may include cerclage applications such as in trochanteric fixation in addition to application of a substantially rigid tether to multiple spinous processes or lamina. Figs. 6A-6B illustrate the use of a tether and fastening mechanism for trochanteric fixation. Fig. 6A shows a tether T wrapped around the tronchanter of a femur F. A fastening mechanism FM releasably locks one end of the tether T, thereby forming a closed loop around the trochanter. Fig. 6B highlights the tether wrapped around the trochanter.

While the above is a complete description of the preferred embodiments of the invention, various alternatives, modifications, and equivalents may be used. Therefore, the above description should not be taken as limiting the scope of the invention which is defined by the appended claims.

## Claims

1. A device for restricting flexion of a spinal segment, said device comprising:
a constraint device having a tether structure (82) and a compliance member (80), wherein the constraint device is adapted to be coupled with adjacent spinous processes or a spinous process and a sacrum, and wherein the constraint device is adapted to provide a force resistant to flexion of the spinal segment; and
a locking mechanism coupled with the constraint device, the locking mechanism comprising a clamp body (84) and a fastener element (81),
wherein the clamp body (84) has a fastener aperture (85) and a tether aperture (87), the fastener aperture being sized to receive the fastener element; and the tether aperture being sized to receive the tether structure, and
wherein a portion of the tether structure (82) is disposed in both the tether aperture (87) and the fastener aperture (85), and
wherein the fastener element (81) is disposed in the fastener aperture (85) such that the tether structure (82) is captured between the fastener element (81) and the clamp body (84).

2. The device of claim 1, wherein the tether aperture (87) comprises a rectangular shaped slot.

3. The device of claim 1, wherein the tether structure (82) enters the tether aperture (87) in a first plane and the tether structure (82) exits the fastener aperture (85) in a second plane transverse to the first plane.

4. The device of claim 1, wherein the fastener element (81) comprises a screw threadably engaged with the clamp body (84).

5. The device of claim 4, wherein the screw (81) comprises a screw head and wherein the tether structure (82) is clamped between the screw head and the clamp body (84).

6. The device of claim 1, wherein the tether structure (82) enters the tether aperture (87) without being deformed.

7. The device of claim 1, wherein the tether structure (82) has a width and the tether aperture (87) has a width smaller than the tether width.

8. The device of claim 1, wherein a surface of the clamp body (84) comprises surface features adapted to press into the tether structure (82) when the fastener element (81) is attached to the clamp body (84).

9. The device of claim 1, wherein the constraint provides an elastic resistance to flexion of the spinal segment.

10. The device of claim 9, wherein the elastic resistance is in the range from 7.5 - 20 N/mm.

11. The device of claim 1, wherein the constraint is configured to couple two spinous processes together.

12. The device of claim 1, wherein the fastener element (81) comprises a driver feature.

## Patentansprüche

1. Vorrichtung zum Beschränken einer Beugung eines Wirbelsäulensegments, wobei die Vorrichtung Folgendes umfasst:
eine Beschränkungsvorrichtung, die eine Haltebandstruktur (82) und ein Nachgiebigkeitselement (80) aufweist, wobei die Beschränkungsvorrichtung dafür ausgelegt ist, mit benachbarten Dornfortsätzen oder einem Dornfortsatz und einem Kreuzbein gekoppelt zu werden, und wobei die Beschränkungsvorrichtung dafür ausgelegt ist, einen Kraftwiderstand gegen eine Beugung des Wirbelsäulensegments bereitzustellen und
einen Verriegelungsmechanismus, der mit der Beschränkungsvorrichtung gekoppelt ist, wobei der Verriegelungsmechanismus einen Klemmkörper (84) und ein Befestigungselement (81) umfasst,
wobei der Klemmkörper (84) eine Befestigungsöffnung (85) und eine Haltebandöffnung (87) aufweist, wobei die Befestigungsöffnung so bemessen ist, dass sie das Befestigungselement aufnimmt; und wobei die Haltebandöffnung so bemessen ist, dass sie die Haltebandstruktur aufnimmt,
und wobei ein Abschnitt der Haltebandstruktur (82) sowohl in der Haltebandöffnung (87) als auch in der Befestigungsöffnung (85) angeordnet ist,
und wobei das Befestigungselement (81) so in der Befestigungsöffnung (85) angeordnet ist, dass die Haltebandstruktur (82) zwischen dem Befestigungselement (81) und dem Klemmkörper (84) eingefangen wird.

2. Vorrichtung nach Anspruch 1, wobei die Haltebandöffnung (87) einen rechteckig geformten Schlitz umfasst.

3. Vorrichtung nach Anspruch 1, wobei die Haltebandstruktur (82) in einer ersten Ebene in die Haltebandöffnung (87) eintritt und die Haltebandstruktur (82) aus der Befestigungsöffnung (85) in einer zweiten Ebene quer zur ersten Ebene austritt.

4. Vorrichtung nach Anspruch 1, wobei das Befestigungselement (81) eine Schraube umfasst, die über ein Gewinde mit dem Klemmkörper (84) in Eingriff steht.

5. Vorrichtung nach Anspruch 4, wobei die Schraube (81) einen Schraubenkopf umfasst und wobei die Haltebandstruktur (82) zwischen dem Schraubenkopf und dem Klemmkörper (84) eingeklemmt ist.

6. Vorrichtung nach Anspruch 1, wobei die Haltebandstruktur (82) in die Haltebandöffnung (87) eintritt, ohne verformt zu werden.

7. Vorrichtung nach Anspruch 1, wobei die Haltebandstruktur (82) eine Breite aufweist und die Haltebandöffnung (87) eine Breite aufweist, die kleiner als die Breite des Haltebands ist.

8. Vorrichtung nach Anspruch 1, wobei eine Oberfläche des Klemmkörpers (84) Oberflächenmerkmale umfasst, die geeignet sind, in die Haltebandstruktur (82) zu drücken, wenn das Befestigungselement (81) an dem Klemmkörper (84) befestigt ist.

9. Vorrichtung nach Anspruch 1, wobei die Beschränkung einen elastischen Widerstand gegen die Beugung des Wirbelsäulensegments bereitstellt.

10. Vorrichtung nach Anspruch 9, wobei der elastische Widerstand im Bereich von 7,5 bis 20 N/mm liegt.

11. Vorrichtung nach Anspruch 1, wobei die Beschränkung so konfiguriert ist, dass sie zwei Dornfortsätze miteinander koppelt.

12. Vorrichtung nach Anspruch 1, wobei das Befestigungselement (81) eine Treiberfunktion umfasst.

## Revendications

1. Dispositif permettant de limiter la flexion d'un segment médullaire, ledit dispositif comportant :
un dispositif de contrainte ayant une structure d'attache (82) et un élément d'élasticité (80), dans lequel le dispositif de contrainte est adapté à des fins d'accouplement avec des apophyses épineuses adjacentes ou une apophyse épineuse et un os sacrum, et dans lequel le dispositif de contrainte est adapté à des fins de mise en oeuvre d'une force destinée à résister à la flexion du segment médullaire ; et
un mécanisme de verrouillage accouplé au dispositif de contrainte, le mécanisme de verrouillage comportant un corps de serrage (84) et un élément de fixation (81),
dans lequel le corps de serrage (84) a une ouverture (85) pour élément de fixation et une ouverture (87) pour structure d'attache, l'ouverture pour élément de fixation étant dimensionnée afin de recevoir l'élément de fixation ; et l'ouverture pour structure d'attache étant dimensionnée afin de recevoir la structure d'attache, et
dans lequel une partie de la structure d'attache (82) est disposée dans à la fois l'ouverture (87) pour structure d'attache et l'ouverture (85) pour élément de fixation, et
dans lequel l'élément de fixation (81) est disposé dans l'ouverture (85) pour élément de fixation de telle sorte que la structure d'attache (82) est capturée entre l'élément de fixation (81) et le corps de serrage (84).

2. Dispositif selon la revendication 1, dans lequel l'ouverture (87) pour structure d'attache comporte une fente de forme rectangulaire.

3. Dispositif selon la revendication 1, dans lequel la structure d'attache (82) entre dans l'ouverture (87) pour structure d'attache dans un premier plan et la structure d'attache (82) sort de l'ouverture (85) pour élément de fixation dans un second plan transversal par rapport au premier plan.

4. Dispositif selon la revendication 1, dans lequel l'élément de fixation (81) comporte une vis mise en prise par filetage avec le corps de serrage (84).

5. Dispositif selon la revendication 4, dans lequel la vis (81) comporte une tête de vis et dans lequel la structure d'attache (82) est serrée entre la tête de vis et le corps de serrage (84).

6. Dispositif selon la revendication 1, dans lequel la structure d'attache (82) entre dans l'ouverture (87) pour structure d'attache sans subir de déformation.

7. Dispositif selon la revendication 1, dans lequel la structure d'attache (82) a une largeur et l'ouverture (87) pour structure d'attache a une largeur inférieure à la largeur de la structure d'attache.

8. Dispositif selon la revendication 1, dans lequel une surface du corps de serrage (84) comporte des organes de surface adaptés à des fins de compression dans la structure d'attache (82) quand l'élément de fixation (81) est attaché au corps de serrage (84).

9. Dispositif selon la revendication 1, dans lequel la contrainte procure une résistance élastique à la flexion du segment médullaire.

10. Dispositif selon la revendication 9, dans lequel la résistance élastique est de l'ordre de 7,5 à 20 N/mm.

11. Dispositif selon la revendication 1, dans lequel la contrainte est configurée pour accoupler deux apophyses épineuses ensemble.

12. Dispositif selon la revendication 1, dans lequel l'élément de fixation (81) comporte un organe d'entraînement.
